# EUROPÄISCHE PATENTANMELDUNG

(11) **EP 1 121 924 A2**
(43) Veröffentlichungstag der Anmeldung: **08.08.2001**
(21) Anmeldenummer: 01102610.1
(22) Anmeldetag: 06.02.2001
(51) Int. Cl.: A61K 6/00

(54) **Primer zur Vorbereitung bearbeiteter Zahnflächen für Kompositfüllungen und Werkstoffe zum Festsetzen von Zahnersatz**

(30) Priorität: 07.02.2000 DE 10005293
(71) Anmelder: MERZ + CO. GmbH & Co., 60318 Frankfurt (DE)
(72) Erfinder: Dziuron, Peter Dr., 24327 Blekendorf (DE)
(74) Vertreter: Glawe, Delfs, Moll & Partner

(57) **Zusammenfassung**

Gegenstand der Erfindung ist ein Mittel zum Vorbereiten einer Zahnfläche zur Verbindung mit einem dentalen Komposit- oder Befestigungsmaterial. Erfindungsgemäß ist vorgesehen, daß das Mittel aminfrei ist und in wäßriger Lösung enthält: a) Phosphorsäure und/oder Phosphorsäureester und/oder Diphosphate, b) einen in saurem Milieu wirksamen Elektronendonator ausgewählt aus der Gruppe bestehend aus Sulfinsäuren und deren Salzen. Der erfindungsgemäß vorgesehene Elektronendonator ersetzt das in saurem Milieu unwirksame Amin eines Amin/Peroxid Autopolymerisationsinitiationssystems. Das erfindungsgemäße Mittel vermittelt daher auch bei der Verwendung von autopolymerisierenden Bond- bzw. Kompositmaterialsystemen eine hohe Haftung zwischen Zahn und Komposit- oder Ersatz- bzw. Befestigungsmaterial.

## Beschreibung

Die Erfindung betrifft ein Mittel zum Vorbereiten einer Zahnfläche oder Zahnkavität zur Verbindung mit einem dentalen Kompositmaterial, insbesondere zur Vorbereitung bearbeiteter Zahnflächen für Kompositfüllungen und Werkstoffe zum Festsetzen von Zahnersatz, sowie einen Kit zur Herstellung eines solchen Mittels.

Vor dem Füllen von Zahnkavitäten mit einem polymerisierbaren Kompositmaterial auf Kunststoffbasis muß die Zahnsubstanz (Dentin oder Schmelz) vorbehandelt werden, um eine gute Haftung des Komposits daran sicherzustellen. Ein guter Verbund zwischen Zahn und Füllung ist wichtig, da alle bekannten Kompositmaterialien insbesondere in der Anfangsphase der Polymerisation schrumpfen. Bei mangelnder Haftung kommt es zu einer Randspaltbildung, in den Randspalt können Bakterien eindringen und Sekundärkaries hervorrufen und/oder die Pulpa schädigen.

Aus JP-A-7082115 sind Primer bekannt, die fünf wesentliche Bestandteile (Vinylverbindungen mit einer sauren Gruppe, Vinylverbindungen mit einer Hydroxylgruppe, Wasser, aromatische Sulfinsäuresalze und aromatische Amine) in einem bestimmten Verhältnis enthalten. Sämtliche Bestandteile werden als wesentlich zur Erzielung einer hinreichenden Haftfestigkeit beschrieben.

Aus DE 196 51 121 A1 ist ein eingangs genannter Primer bekannt. Der Erfindung liegt die Aufgabe zugrunde, ein solches Mittel bzw. einen solchen Primer zu schaffen, der gute Haftwerte auch bei autopolymerisierenden (selbsthärtenden) dentalen Kompositmaterialien aufweist.

Die Erfindung löst diese Aufgabe dadurch, daß das Mittel aminfrei ist und in wäßriger Lösung enthält:
a) Phosphorsäure und/oder Phosphorsäureester und/oder Diphosphate,
b) einen in saurem Milieu wirksamen Elektronendonator ausgewählt aus der Gruppe bestehend aus Sulfinsäuren und deren Salzen.
Zunächst seien einige Begriffe erläutert.

"Aminfrei" bedeutet, daß die üblicherweise zusammen mit einem Peroxid als Polymerisationsinitiatoren eingesetzten Amine in dem erfindungsgemäßen Mittel nicht oder allenfalls in einem sehr geringen, durch etwaige Verunreinigungen hervorgerufenen Anteil (vorzugsweise unter 0,5 Gew.-%) enthalten sind. Der Begriff "aminfrei" schließt nicht aus, daß Amine in den zusätzlich zu dem erfindungsgemäßen Mittel möglicherweise eingesetzten Bonds oder Kompositmaterialien enthalten sind oder als separate Polymerisationsinitiatoren bei der Verarbeitung des erfindungsgemäßen Primers zusammen mit solchen Bonds oder Kompositmaterialien zugesetzt werden.

Im Rahmen der Erfindung umfaßt der in Anspruch 1 verwendete Begriff "Vorbereitung einer Zahnfläche zur Verbindung mit einem dentalen Kompositmaterial" sowohl das Ätzen der Zahnfläche als auch das Primen. Das Primen beinhaltet die Herstellung einer guten Verbindung zwischen Primer und Zahnoberfläche, z. B. durch Eindringen der an Spacerabschnitten der Moleküle angeordneten Phosphorsäureenden in die Dentintubuli der Zahnsubstanz. Der Primer bewirkt eine gute mechanische Adhäsion an der Zahnfläche. Die Schritte des Ätzens und Primens können erfindungsgemäß in einem einzigen Schritt ausgeführt werden. Vorzugsweise umfaßt der genannte Begriff zusätzlich auch das sogenannte Bonding. Mit Bonding bezeichnet man das Schaffen einer guten Haftgrundlage für das anschließende Einpolymerisieren des Kompositmaterials.

Der Begriff Phosphorsäureester umfaßt sowohl Mono-, Di- als auch Triester der Phosphorsäure bzw. von deren Salzen. Als Alkoholkomponente der Ester wird erfindungsgemäß vorzugsweise Hydroxypropylmethacrylat (HPMA) verwendet, die Phosphorsäureester sind also bevorzugt HPMA-Ester. Andere geeignete Alkoholgruppen sind insbesondere bevorzugt wasserlösliche Vinylverbindungen mit einer Hydroxylgruppe. Erfindungsgemäß ist es jedoch bevorzugt, wenn das Mittel kein Hydroxyethylmethacrylat (HEMA) bzw. HEMA-Ester enthält. Erfindungsgemäß kann somit auf den Einsatz dieser ein hohes Allergierisiko aufweisenden Substanz verzichtet werden.

Zwar offenbart bereits JP-A-7082115 den Zusatz aromatischer Sulfinsäuresalze zu einer im Dentalbereich verwendbaren Grundiermischung. Jedoch wird ausdrücklich gesagt, daß dies immer nur in Kombination mit einem aromatischen sekundären und/oder tertiären Amin geschehen kann, der Amingehalt der Mischung muß dieser JP-Schrift zufolge zwischen 2 und 15 Gew.-% liegen. Es heißt weiter, daß die Klebefestigkeit steigernde Wirkung bei einer Aminkonzentration außerhalb dieses Bereichs gravierend absinkt. Ferner hat dieser Stand der Technik den Nachteil, daß Phosphatester mit verhältnismäßig langen Estergruppen eingesetzt werden, die keine hinreichende Wassermischbarkeit aufweisen, so daß das allergene HEMA nicht nur zur Erzielung der Haftung, sondern in erheblichen Mengen auch als Lösungsvermittler zugesetzt werden muß. Die JP-Schrift bezeichnet jede der fünf in der Beschreibungseinleitung genannten Stoffgruppen als zwingend notwendig für die Erzielung hinreichender Scherhaftfestigkeit an Dentin, sie offenbart damit ein komplexes, schwierig zu formulierendes Vielstoffsystem, das insbesondere durch den Amingehalt zudem Probleme mit der Lagerstabilität haben kann. Erfindungsgemäß wird demgegenüber ein einfach zu formulierendes und handhabendes Primersystem geschaffen, bei dem lediglich drei Bestandteile (Phosphatester, Sulfinsäure, Wasser) zwingend erforderlich sind.

Unter einem in sauren Milieu wirksamen Elektronendonator ist jede Substanz zu verstehen, die bei pH-Werten unter 7, bevorzugt auch im stark sauren Bereich (pH 3 und kleiner), mit einem Polymerisationsinitiator eines autopolymerisierenden Kompositmaterials oder Bondes in einer Art und Weise reagieren kann, daß der erste, zur Bildung von Radikalen notwendige Reaktionsschritt am Peroxid abläuft. Um diese Radikalbildung zu vollziehen, muß es zu einem Elektronenübergang auf den Peroxidsauerstoff kommen. Der erfindungsgemäß verwendete Elektronendonator sorgt dafür, daß dieser erste Schritt auch in dem durch Phosphorsäure geschaffenen stark sauren Milieu funktionieren kann. Der Sulfinatgehalt liegt vorzugsweise zwischen 1 und 10 Gew.-%, und weiter vorzugsweise beträgt er 2 bis 5 Gew.-%, besonders bevorzugt etwa 3 Gew.-%. Dieser Gehalt wird berechnet als Natriumsulfinat.

Die Erfindung hat erkannt, daß ein Primer des Standes der Technik wie beispielsweise aus DE 196 51 121 A1 mit einem autopolymerisierenden Kompositmaterial nicht oder allenfalls eingeschränkt verwendbar ist. In einem solchen Kompositmaterial soll die Polymerisation zur Reaktion in der Regel dadurch gestartet werden, daß ein Peroxid wie beispielsweise Benzoylperoxid zur Radikalbildung angeregt wird. Zu diesem Zweck ist in der Regel ein Amin wie beispielsweise N,N-dimethyl-p-toluidin vorgesehen, dessen freies Elektronenpaar am Aminstickstoff mit dem Peroxid wechselwirken und so die Radikalbildungsreaktion in Gang setzen soll. Die Erfindung hat erkannt, daß diese Radikalbildungsreaktion bei Kontakt mit einem phosphorsauren Primer nicht oder allenfalls unzureichend funktioniert, da sich in diesem sauren Milieu ein Proton an das freie Elektronenpaar des Aminstickstoffs anlagert und so dessen Elektronendonorfunktion unterbindet.

Erfindungsgemäß ist daher der Zusatz eines auch in (stark) sauren Milieu wirksamen Elektronendonators vorgesehen, so daß die Vorteile autopolymerisierender Systeme auch in Verbindung mit einem phosphorsauren Primer (Mittel zum Vorbereiten einer Zahnfläche) nutzbar gemacht werden können. Dieser Elektronendonator ist vorzugsweise Bestandteil des Primers und ersetzt und/oder ergänzt daher unter Umständen einen bereits im dentalen Kompositmaterial oder Bond vorgesehenen Elektronendonator, der durch den Kontakt mit dem phosphorsauren Primer ganz oder teilweise unwirksam gemacht wird.

Häufig werden Primer gemäß der Erfindung erst kurz vor der Verwendung aus einem Zweikomponentenkit angesetzt. Es ist dann besonders bevorzugt, wenn eine Komponente Phosphorsäure und/oder Phosphorsäureester und/oder Diphosphate enthält und die andere Komponente in wäßriger Lösung ein Salz einer Sulfinsäure. Sulfinate haben gegenüber der freien Sulfinsäure den Vorteil der Beständigkeit in wäßriger Lösung, jedoch ist bei den Salzen das freie Elektronenpaar des Schwefels stärker an dieses Atom gebunden und steht somit für die Initialisierung des Peroxids nur eingeschränkt zur Verfügung. Erfindungsgemäß wird aus dem Zweikomponentenkit kurz vor der Verwendung ein erfindungsgemäßes Mittel hergestellt. Durch das phosphorsaure Milieu der ersten Komponente (Komponente A) bildet sich dann freie Sulfinsäure, die ein wirksamer Elektronendonator ist. Der Nachteil der freien Sulfinsäure, nämlich die leichte Oxidierbarkeit bzw. eine Disproportionierung, fällt bei dem Freisetzen dieser freien Säure kurz vor der Anwendung nicht ins Gewicht.

Erfindungsgemäß wird somit ein Primer geschaffen, der auch zusammen mit autopolymerisierenden Kompositmaterialien die in DE 196 51 121 A1 beschriebenen Vorteile erzielt, insbesondere die Vorbehandlung der Zahnfläche in einem einzigen Schritt und die Vermittlung einer hohen Haftung zwischen Zahnfläche und dem aufgebrachten Kompositmaterial. Der erfindungsgemäß zugesetzte, im sauren Milieu wirkende Elektronendonator ersetzt einen ggf. entweder im aufgebrachten Bond oder aufgebrachten Kompositmaterial enthaltenen Aminstarter, der durch das saure Milieu, wie oben beschrieben, unwirksam wird. Durch Radikalbildung des Peroxids mittels des Elektronendonators, wie beispielsweise der Sulfinsäure wird die Autopolymerisationsreaktion dennoch in Gang gebracht.

Das erfindungsgemäße Mittel enthält besonders bevorzugt:
a) 1 - 5 Gew.-% Phosphorsäure;
b) 10 - 90 Gew.-% Phosphorsäuremono- und/oder -diester der Formel 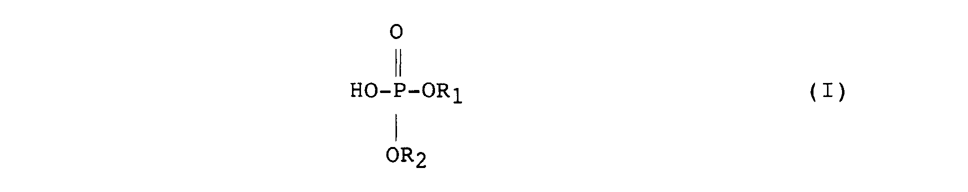 wobei
   - R₁: gleich 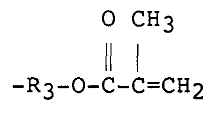 ist;
   - R₂: gleich H oder 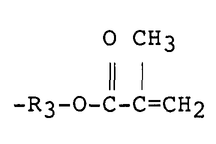 ist;
   - R₃: ausgewählt ist aus der Gruppe bestehend aus Alkylengruppen, Alkylenoxygruppen mit einer oder mehreren Alkylenoxyeinheiten, mit einer oder mehreren Hydroxygrupper substituierten Alkylen- oder Alkylenoxygruppen;
c) 1 - 15 Gew.-% Phosphorsäuretriester der Formel

   O=P(OR₁)₃ (II)

   mit der oben angegebenen Bedeutung für R₁;
d) 3 - 50 Gew.-% Diphosphate der Formel 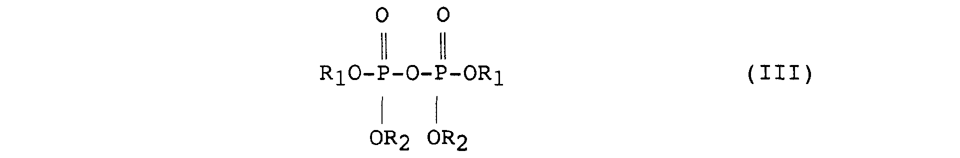 mit den oben angegebenen Bedeutungen für R₁ und R₂, wobei R₂ in Formel (III) gleich oder verschieden sein kann.

Das erfindungsgemäße Mittel enthält eine Mischung aus Phosphorsäure, einem Hauptanteil Phosphorsäuremono- und/oder diestern, ferner Phosphorsäuretriester und Diphosphate. Der geringe Phosphorsäureanteil dient dem vorbereitenden Ätzen der Zahnfläche. Die erfindungsgemäß verwendeten Phosphorsäureester sind Moleküle, deren saure Hydroxygruppen sich chemisch mit dem Kalzium der Zahnoberfläche verbinden können. Die teilveresterte Phosphorsäuregruppe ist über eine als Spacer dienende Alkylen- oder Alkylenoxygruppe (die ggf. Hydroxygruppen trägt) mit einer Methacrylatgruppe verbunden. Die Methacrylatgruppen können teilweise polymerisieren und so einen Film auf der Zahnoberfläche bilden, in die das Kompositmaterial einpolymerisieren kann. Die Phosphorsäureenden der Moleküle können aufgrund ihrer guten Beweglichkeit in Dentintubuli eindringen und so die mechanischen Adhäsion an der Zahnoberfläche verbessern.

Die Prozentangaben in Anspruch 3 beziehen sich auf den sog. Harzanteil der Mischung, der bei einem lösemittelfreien Gemisch 100 % beträgt. Er verringert sich entsprechend der zugesetzten Wassermenge.

Es hat sich gezeigt, daß die erfindungsgemäße Mischung von Phosphorsäure, -mono-, di- und triestern sowie Diphosphaten sowohl über eine gute Lagerstabilität als auch eine sehr hohe Haftkraft verfügt. Ätzen, Primen und ggf. Bonding lassen sich mit dieser Mischung in einem einzigen Verfahrensschritt durchführen.

Die Spacergruppen in den Phosphorsäurederivaten wird vorzugsweise ausgewählt aus der Gruppe bestehend aus Alkylengruppen mit 2-4 C-Atomen, Ethylenoxy- oder Propylenoxygruppen, die aus bis zu 4 Glykoleinheiten (Ethylen- oder Propylenglykoleinheiten) bestehen können, sowie Alkylengruppen mit 2-4 C-Atomen, die mit einer oder zwei Hydroxygruppen substituiert sind.

Bevorzugt als Spacer sind Ethylen- oder Propylengruppen, ferner eine Gruppe der Formel 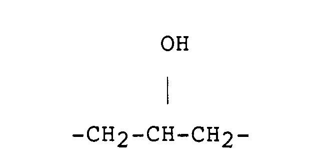 oder ein Isomeres davon.

In der Mischung besteht der Hauptanteil vorzugsweise aus Phosphorsäuremono- und diestern, er macht vorzugsweise 30 - 90 Gew.-%, weiter vorzugsweise 40 - 80 Gew.-% bezogen auf den Harzanteil aus. Weiter bevorzugt ist ein Verhältnis von 20 - 50 Gew.-% Phosphorsäuremonoester und 25 - 45 Gew.-% Phosphorsäurediester. Der Phosphorsäuregehalt der Mischung beträgt vorzugsweise 1 - 3 Gew.-%.

Die in Anspruch 1 und den Unteransprüchen enthaltene Aufzählung möglicher Bestandteile des erfindungsgemäßen Mittels ist nicht abschließend, weitere Bestandteile wie beispielsweise polymerisierbare Säuren, Polymerisationsinitiatoren und dergleichen können darin bei Bedarf enthalten sein.

Der Wasseranteil an der angesetzten, verwendungsfertigen Mischung beträgt vorzugsweise 20 - 95 Gew.-%, weiter vorzugsweise 20 - 50 Gew.-%, besonders bevorzugt 25 - 30 Gew.-%. Niedrige Wassergehalte von maximal 50, vorzugsweise maximal 40, weiter vorzugsweise maximal 30 Gew.-% sind bevorzugt, was aufgrund der bei niedrigerem Wassergehalt höheren Viskosität des erfindungsgemäßen Mittels die Applikation im Mundbereich wesentlich erleichtert.

Gegenstand der vorliegenden Erfindung ist ferner die Verwendung eines vorstehend beschriebenen Mittels zur Herstellung eines Primers zur Vorbereitung einer Zahnfläche bzw. Zahnkavität zur Verbindung mit einem dentalen Kompositmaterial.

Der erfindungsgemäße Primer kann auch zusammen mit lichthärtenden Komposit- bzw. Bondmaterialien verwendet werden. In diesem Fall übt der erfindungsgemäß zugesetzte Elektronendonator keinerlei Wirkung aus und hat insbesondere keine unerwünschten Nebenwirkungen. Das erfindungsgemäße Mittel kann somit universell mit auto- sowie lichthärtenden Bond-, Befestigungs- bzw. Kompositmaterialien verwendet werden und vermittelt in allen Fällen eine hohe Haftung zwischen Zahn und Kompositmaterial.

Die Erfindung wird nachfolgend anhand eines Ausführungsbeispiels und eines Vergleichsbeispiels erläutert.

### Beispiel 1:

29 g HPMA werden in 50 ml Chloroform aufgenommen. Zur Lösung werden unter Rühren portionsweise 15 g Phosphorpentoxid gegeben. Nach 24h wird filtriert, mit 50 ml Wasser versetzt und weitere 24h gerührt. Danach wird die Chloroformphase abgetrennt und unter Vakuum eingeengt. Ausbeute 32 g. Die Reaktionsmischung wird mittels ³¹P-NMR-Spektroskopie analysiert. Sie hat folgende Zusammensetzung: 1 % Phosphorsäure, 39,5 % Phosphorsäuremonoester, 29,1 % Phosphorsäurediester, 0,5 % Phosphorsäuretriester, 28,5 % Diphosphat. Sämtliche Prozentangaben sind Gewichtsprozente.

### Beispiel 2:

Sofern man einen lichthärtenden Primer herstellen will, werden 25 g der Reaktionsmischung aus Beispiel 1 mit 0,1 g Campherchinon versetzt.

### Beispiel 3:

1 g der Mischung aus Beispiel 1 oder 2 wird mit 1 g einer wäßrigen 3%igen Natriumsulfinatlösung kurz vermischt und direkt für Haftwertversuche nach dem in ISO-Entwurf TF 11405 beschriebenen Verfahren eingesetzt. Die Haftwerte, die ein autopolymerisierendes Kompositmaterial (Degufill® SC Bond und Degufill® SC) auf Rinderdentin unter Verwendung der Mischung des Beispiels 1 hat, ergeben im Durchschnitt 21,3 MPa.

### Vergleichsbeispiel

Bei Einsatz der Mischung aus dem Beispiel 1 sowie Verdünnung dieser Lösung mit einem wäßrigen Lösungsmittel analog Beispiel 3, jedoch ohne Zusatz von Natriumsulfinat ergeben bei analogen Haftwertversuchen lediglich Werte zwischen 5 und 7 MPa.

## Patentansprüche

1. Mittel zum Vorbereiten einer Zahnfläche zur Verbindung mit einem dentalen Kompositmaterial, dadurch gekennzeichnet, daß es aminfrei ist und in wäßriger Lösung enthält:
a) Phosphorsäure und/oder Phosphorsäureester und/oder Diphosphate,
b) einen in saurem Milieu wirksamen Elektronendonator ausgewählt aus der Gruppe bestehend aus Sulfinsäuren und deren Salzen.

2. Mittel nach Anspruch 1, dadurch gekennzeichnet, daß es kein Hydroxyethylmethacrylat (HEMA) enthält.

3. Mittel nach Anspruch 1 oder 2, dadurch gekennzeichnet, daß es Hydroxypropylmethacrylat (HPMA) enthält.

4. Mittel nach einem der Ansprüche 1 bis 3, dadurch gekennzeichnet, daß es enthält:
a) 1 - 5 Gew.-% Phosphorsäure;
b) 10 - 90 Gew.-% Phosphorsäuremono- und/oder -diester der Formel 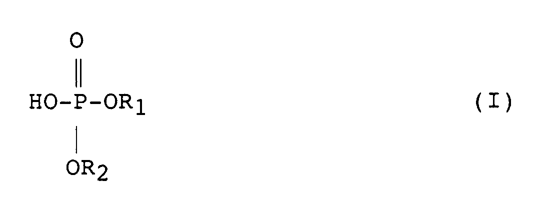 wobei
R₁ gleich 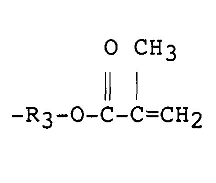 ist;
R₂ gleich H oder 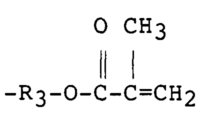 ist;
R₃ ausgewählt ist aus der Gruppe bestehend aus Alkylengruppen, Alkylenoxygruppen mit einer oder mehreren Alkylenoxyeinheiten, mit einer oder mehreren Hydroxygruppen substituierten Alkylen- oder Alkylenoxygruppen;
c) 1 - 15 Gew.-% Phosphorsäuretriester der Formel
O=P(OR₁)₃ (II)
mit der oben angegebenen Bedeutung für R₁;
d) 3 - 50 Gew.-% Diphosphate der Formel 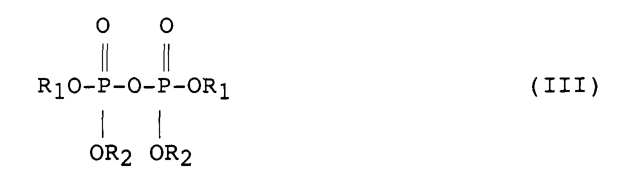 mit den oben angegebenen Bedeutungen für R₁ und R₂, wobei R₂ in Formel (III) gleich oder verschieden sein kann.

5. Mittel nach Anspruch 4, dadurch gekennzeichnet, daß R₃ ausgewählt ist aus der Gruppe bestehend aus Alkylengruppen mit 2 - 4 C-Atomen, Ethylenoxy- oder Propylenoxygruppen aus bis zu 4 Glykoleinheiten, Alkylengruppen mit 2 - 4 C-Atomen, die mit einer oder zwei Hydroxygruppen substituiert sind.

6. Mittel nach Anspruch 5, dadurch gekennzeichnet, daß R₃ eine Ethylen- oder Propylengruppe ist.

7. Mittel nach Anspruch 5, dadurch gekennzeichnet, daß R₃ gleich 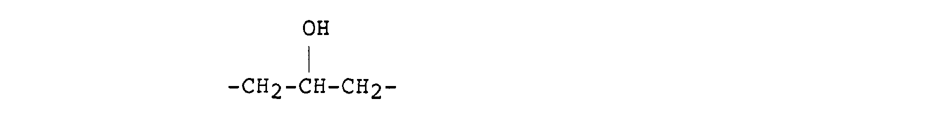 oder ein Isomeres davon ist.

8. Mittel nach einem der Ansprüche 4 bis 7, dadurch gekennzeichnet, daß der Anteil der Phosphorsäuremono- und/oder -diester an der Mischung 30 - 90 Gew.-%, vorzugsweise 40 - 80 Gew.-% beträgt.

9. Mittel nach Anspruch 8, dadurch gekennzeichnet, daß die Mischung 20 - 50 Gew.-% Phosphorsäuremonoester und 25 - 45 Gew.-% Phosphorsäurediester enthält.

10. Mittel nach einem der Ansprüche 4 bis 9, dadurch gekennzeichnet, daß die Mischung 1 - 3 Gew.-% Phosphorsäure enthält.

11. Mittel nach einem der Ansprüche 1 bis 10, dadurch gekennzeichnet, daß der Anteil des Lösungsmittels Wasser an der gesamten Mischung 20 - 95 Gew.-%, vorzugsweise 20 - 50 Gew.-%, weiter vorzugsweise 25 - 30 Gew.-% beträgt.

12. Verwendung eines Mittels nach einem der Ansprüche 1 bis 11 zur Herstellung eines Primers zur Vorbereitung einer Zahnfläche zur Verbindung mit einem dentalen Kompositmaterial.

13. Kit zur Herstellung eines Mittels nach einem der Ansprüche 1 bis 11, dadurch gekennzeichnet, daß er zwei Komponenten A) und B) aufweist, die jeweils folgende Bestandteile enthalten:
Komponente A): Phosphorsäure und/oder Phosphorsäureester
und/oder Diphosphate;
Komponente B): ein Salz einer Sulfinsäure in wäßriger Lösung.
